# EUROPEAN PATENT SPECIFICATION

(11) **EP 1 226 871 B1**
(45) Date of publication and mention of the grant of the patent: **16.08.2006**
(21) Application number: 02001708.3
(22) Date of filing: 24.01.2002
(51) Int. Cl.: B01L 3/00, C12Q 1/68, G01N 33/543, G01N 33/552

(54) **Reactive probe chip and fluorescence detection sytem**
Reaktiver Sondenchip und Fluoreszenz-Detektionssystem
Sonde-puce réactive et syteme de detection de fluorescence

(30) Priority: 24.01.2001 JP 2001016012
(43) Date of publication of application: 31.07.2002
(73) Proprietor: EBARA CORPORATION, Ohta-ku, Tokyo (JP)
(72) Inventor: Nagasawa, Hiroshi, Hirakata-shi, Osaka (JP)
(74) Representative: Wagner, Karl H.

(56) References cited:
- EP-A- 0 228 075
- WO-A-00/20177
- WO-A-96/03212
- WO-A-99/60170
- DE-U- 20 005 738

## Description

This invention relates to a reaction probe chip, which is used for diagnosis of genes and physiological functions and which enables recognition of many functional molecules, and a detection system for detecting a reaction product obtained by the reaction probe chip.

### Description of the Related Art

Detection of polymorphism due to variation of a gene, especially variation of one base (sequence), is effective for diagnosis of a disease ascribed to mutation or the like, e.g., cancer. Its detection is also necessary for guidelines on drug response and adverse reactions, and contributes to analysis of genes related to causes of multiple factor diseases and contributes to predictive medical care. The use of a so-called DNA chip is known to be effective for this detection.

A hitherto utilized DNA chip having short DNA strands fixed thereto, so-called Gene Chip of Affymetrix, usually comprises 10,000 or more oligo-DNA fragments (DNA probes) fabricated on a silicon or glass substrate 1 cm square by photolithography technology. When a DNA sample, for example, labeled with fluorescence is flowed on this DNA chip, DNA fragments having sequences complementary to the probes on the DNA chip bind to the probes. Only the bound portions can be distinguished by fluorescence, and the particular sequences of the DNA fragments in the DNA sample can be recognized and quantitatively determined. This method has already been shown to be capable of detecting mutation of an oncogene or detecting gene polymorphism.

A microarray having cDNA's arranged on a slide glass is also used. WO 9603212 A1 relates to a multidimensional conduit combinatorial library snythesis device and features methods and devices for synthesizing combinatorial libraries of chemical compounds, in particular for synthesizing N<2> or N<3> compounds. Specifically, a two-dimensional or three-dimensional conduit synthesis device is provided. A multidimensional conduit device for molecular synthesis comprises: (a) a first array of cells aligned along one or more axes; (b) at least a second array of cells aligned along one or more axes; and (c) means for communication among said first and second arrays of cells and to and from each cell in said arrays of cells; and (d) means for moving substrates and reagents among said means for communication. DE 200 05 738 U discloses a densely packed device wherein various target molecules are bonded in a large number of sample areas, wherein carrier plates are used which contain densely packed capillary structures and having a very large inner surface despite small outer dimensions. One thousand times more molecules can be bonded than on a flat outer surface of a comparable size. Cross contamination is avoided by the lack of cross links between the capillaries. Evaporation is minimized by a small outer surface. After the inner surface of the capillaries has been silanized, peptides and peptidomimetics are synthesized in a locally targeted manner. The molecular interactions of components of the substance library with active substances in a solution or a suspension are investigated by means of a local resolution optical detection method. Handling, especially cleaning and covering with substances, is carried out by rinsing liquids through the capillary plate.

The earlier technologies, however, have posed some problems.

For DNA Chip to be produced using photolithography, for example, at least four photomasks are needed for synthesis in one stage, and a cycle of photolithography, coupling and washing has to be repeated four times. Since this procedure is repeated for the necessary strand length, a high cost is involved. In changing patterns, the photomasks need to be changed accordingly. DNA Chip's of various designs, which flexibly meet the needs, have not been available.

DNA Microarray Chip, spotted with a solution of synthetic oligonucleotides at a high density, has been proposed as an alternative method. This type of chip has to undergo a complicated procedure which comprises introducing a modification group in succession to oligonucleotide synthesis, cutting off the modified oligonucleotides from the carrier, followed by detachment and purification, to obtain oligonucleotides, and reacting the oligonucleotides with functional groups introduced onto fixation glass. Thus, this chip is costly, like DNA Chip relying on photolithography.

During detection with these reaction chips, hybridization is localized to lead to a loss in quantitative determinability, and a device dedicated to hybridization should be used and put to a long-term reaction. Thus, detection of various pieces of genetic information, including one for the purpose of bone marrow transplantation, by use of the reaction chips is laborious and time-consuming, and incurs a lot of expenses.

The object of the present invention is to establish a more convenient method for preparing DNA Chip, and to provide a reaction detecting chip and a detection system which can be used in various diagnoses of physiological functions, including DNA polymorphism.

To attain the foregoing object, the present inventors conducted various studies on materials for and shapes of reaction probe chips. Through such studies, they attempted to realize a reaction probe chip, which has on the surface a high integration degree comparable to that obtained by the aforementioned photolithography facilities, without using photolithographic technologies requiring a lengthy, complicated reaction process, posing difficulty in flexibly attaining different objects, and involving huge costs, and without employing a device dedicated to hybridization.

Based on these studies, the inventors found that the above-described problems could be solved by filling and holding carriers into a plurality of through-holes regularly arranged in a substrate, the carriers having different probe molecules fixed for the respective holes, and then introducing a sample into the carrier-filled holes. This finding led them to accomplish the present invention.

The present invention solves the above problems by a reaction probe chip as set forth in claim 1.

Preferred embodiments of the present invention may be gathered from the dependent claims.

Also provided is a reaction product detection system as set forth in claim 6.

According to the present invention, there can be easily provided a reaction detection chip which has reactive substances integrated on its surface, the reactive substances including proteins having arbitrary configurations or oligonucleotides having arbitrary base sequences. The detection chip can be provided without the need for special equipment such as photolithography equipment.

Moreover, the reaction time can be shortened dramatically, the reproducibility of the reaction can be improved, and the total throughput can be increased. Hence, the preparation of a reaction detection chip for DNA or the like, which meets individuals' needs, becomes possible, contributing to made-to-order medical care.

The present invention will become more fully understood from the detailed description given hereinbelow and the accompanying drawings which are given by way of illustration only, and thus are not limitative of the present invention, and wherein:
FIG. 1 is a plan view of a reaction probe chip according to the present invention;
FIGS. 2(a) to 2(d) are partial sectional views showing the state of disposition of various carriers in through-holes of a substrate in the reaction probe chip of the present invention, the carriers in FIGS. 2(a) to 2(d) being a porous substance, a porous membrane, a nonwoven fabric, and a porous glass powder fixed to the membrane or the like, respectively;
FIGS. 3(e) to 3(g) are enlarged partial sectional views illustrating the porous substance, the nonwoven fabric, and the porous glass powder fixed to the nonwoven fabric, respectively;
FIGS. 4(h) and 4(i) are enlarged partial cross-sectional views of the carriers having probe molecules fixed thereto, the carriers being the porous substance in FIG. 4(h) and the nonwoven fabric in FIG. 4(i);
FIG. 5 shows, in cross section, the reaction probe chip of the present invention having the carrier held in the holes, and a stack of the reaction probe chips;
FIGS. 6(j) to 6(1) are sectional explanatory drawings of the reaction probe chip of the present invention fixed in a reaction cell, FIG. 6(j) illustrating the fixed state of the reaction chip in the reaction cell, FIG. 6(k) illustrating the initial state of feeding and suction of a fluorescence labeled sample, and FIG. 6(1) illustrating the distributed state of hybridized spots in the reaction chip;
FIG. 7 is a sectional view illustrating the principle of fluorescence detection showing fluorescence emission from the hybridized spots in the reaction probe chip of the present invention when exposed to ultraviolet radiation;
FIG. 8 is a plan view showing the distributed state of spots upon hybridization of the reaction probe chip of the present invention;
FIG. 9 is a conceptual view of a fluorescence detection system using the reaction probe chip of the present invention; and
FIG. 10 is a schematic drawing of a detection system in which the conceptual view of the fluorescence detection system of the present invention in FIG. 9 is expressed as devices.

Embodiments of the present invention will be described in more detail with reference to the accompanying drawings.

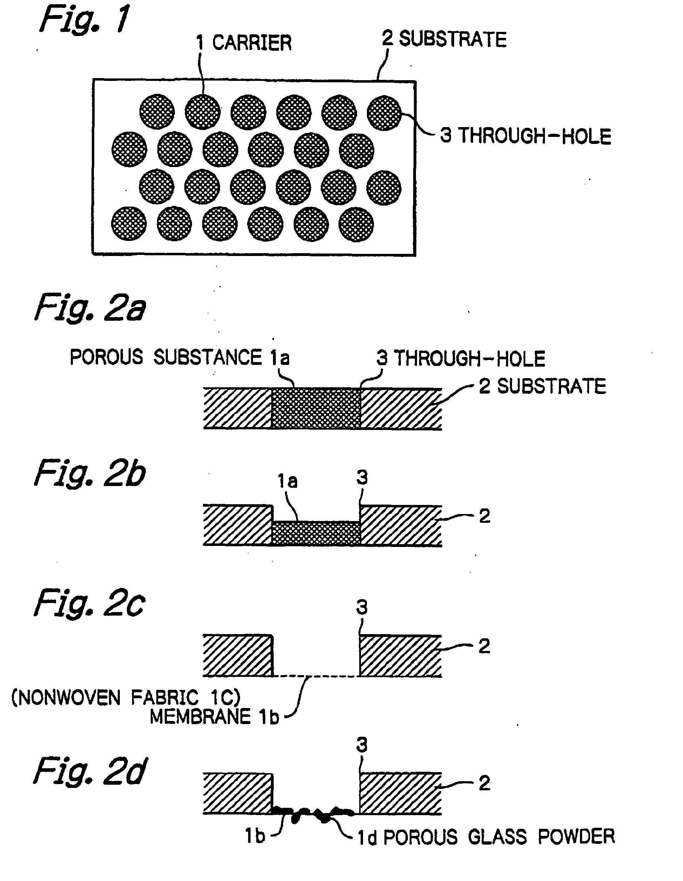

FIG. 1 is an explanatory drawing showing the relationship between a substrate constituting the reaction probe chip of the present invention and a carrier having probe molecules fixed thereto, the substrate having a plurality of discrete through-holes regularly arranged therein. FIG. 1 illustrates, in a plan view, the arranged state of plural through-holes 3 in a substrate 2 which are filled with and hold a carrier 1.

FIGS. 2(a) to 2(d) are partial sectional views showing the state of disposition of various carriers 1 in the through-hole 3 of the substrate 2 in the reaction probe chip shown in FIG. 1. The carriers are liquid permeable, and materials having such properties are collectively called "porous medium or media". Various materials can be used as the porous media. FIG. 2(a) shows a state in which a porous substance 1a as the carrier 1 is filled to the full and held in the through-hole 3. FIG. 2(b) shows a state in which the porous substance 1a is filled in a lower half of the through-hole 3. FIG. 2(c) shows a state in which a porous membrane 1b or a nonwoven fabric 1c, as the carrier 1, is pasted to the lower surface of the through-hole 3. FIG. 2(d) shows a state in which a fine powder of porous glass 1d is further fixed, as a carrier, to the membrane 1b or nonwoven fabric 1c. The porous membrane 1b has liquid permeable micropores, and includes a microporous film.

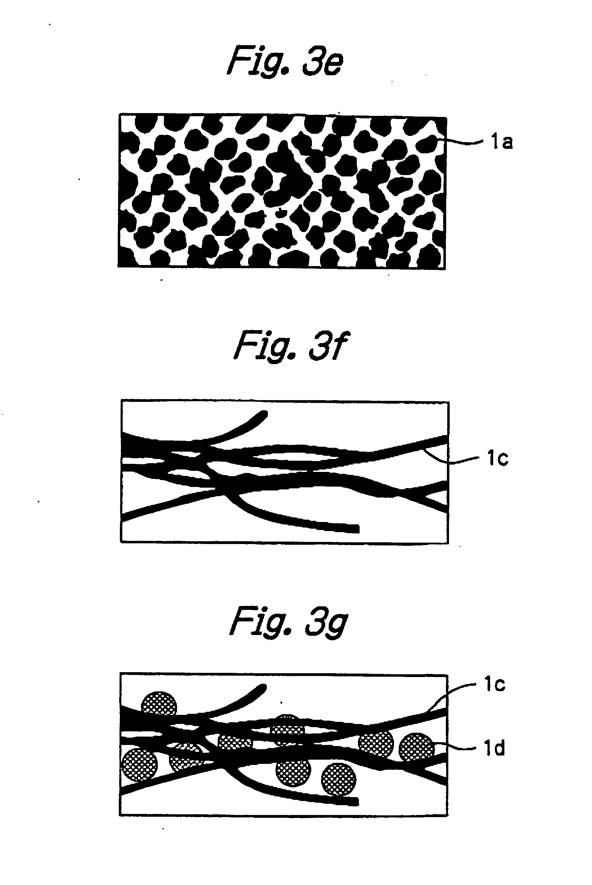

FIGS. 3(e) to 3(g) are enlarged sectional views showing the microstructures of the porous media used in FIGS. 1 and 2(a) to 2(d). In these drawings, black portions represent the solid portions of the porous medium, while white portions represent the porous portions of the porous medium. FIG. 3(e) is an enlarged sectional view of the porous substance 1a as the carrier 1. FIG. 3(f) is an enlarged sectional view of the nonwoven fabric as the carrier 1. FIG. 3(g) is an enlarged sectional view showing the entangled binding of the fine porous glass powder 1d fixed to the fibers of the nonwoven fabric 1c.

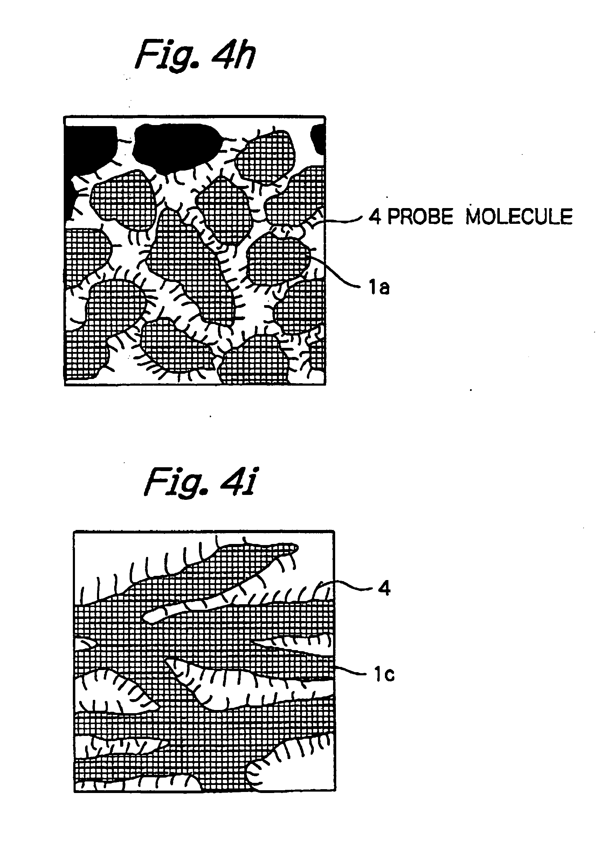

FIGS. 4(h) and 4(i) are enlarged sectional views showing the microstructures of the carriers 1 having probe molecules 4 fixed thereto. FIG. 4(h) illustrates the state of the probe molecules 4 fixed in the porous substance 1a (shaded portions), FIG. 4(i) illustrates the state of the probe molecules 4 fixed to the surfaces of the fibers (shaded portions) of the nonwoven fabric 1c.

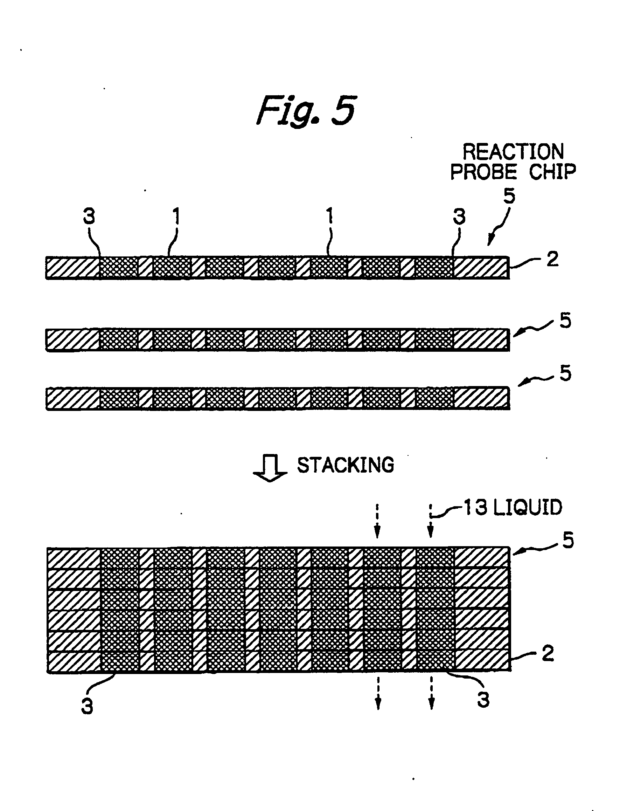

FIG. 5 is a sectional view of the reaction probe chip 5 in which the carrier 1 having different probe molecules 4, 4, and so on fixed thereto is filled and held in a plurality of the through-holes 3, 3, etc. regularly arranged in the substrate 2, along with a sectional view showing a multi-layer stack of the reaction probe chips 5 for simultaneously presenting many of the reaction probe chips 5, 5 ... having the same characteristics.

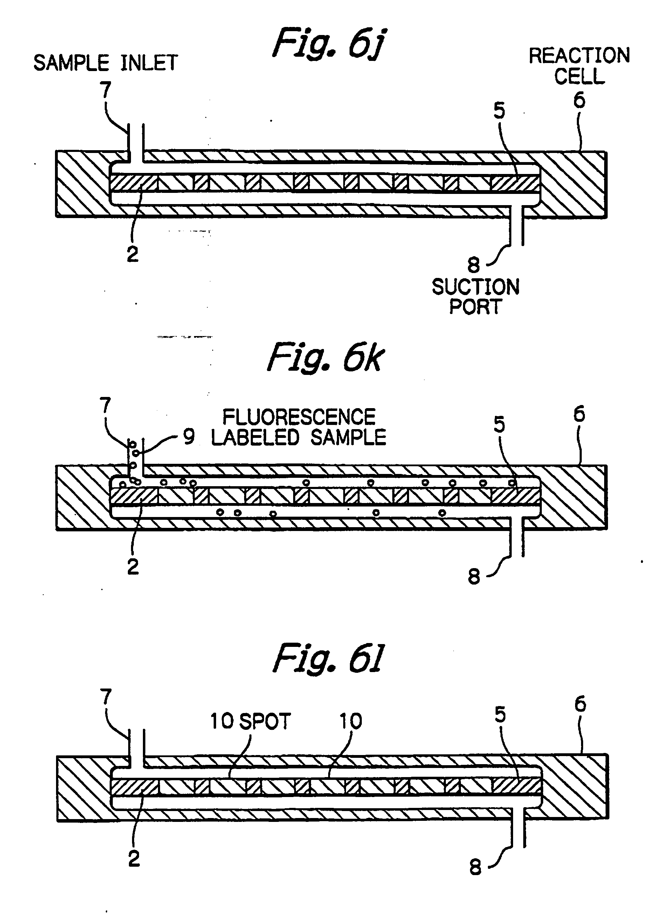

FIGS. 6(j) to 6(1) are sectional views for explaining the status of detection of a sample with the use of the reaction probe chip 5 fixed in a reaction cell 6. FIG. 6(j) is a sectional view showing a state of fixing, in which the reference numeral 7 denotes a sample inlet, 8 denotes a sample suction port, and the other numerals denote portions having the same functions as described earlier. FIG. 6(k) is a sectional view showing a state in which a fluorescence labeled sample 9 begins to be fed and sucked. FIG. 6(1) is a sectional view of hybridized spots 10, 10 upon binding to several types of specific probe molecules 4.

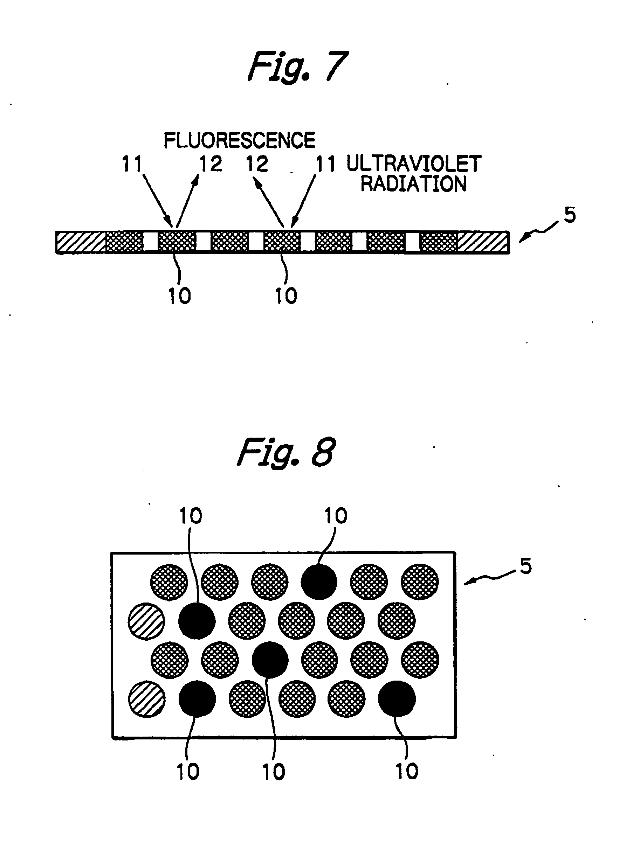

FIGS. 7 and 8 are views illustrating the principle of detection of the hybridized spots 10, 10 by a fluorescence detector. FIG. 7 is a sectional view for explaining a state in which the reaction probe chip 5 having the hybridized spots 10, 10 shown in FIG. 6(1) is irradiated with ultraviolet (UV) radiation 11, and only the spots 10, 10 emit fluorescence 12. FIG. 8 is a plan view showing the distributed state of the through-holes 3 having the hybridized spots 10, 10 among the plural through-holes 3 regularly arranged in the substrate 2. FIG. 8 also shows the locations of emission of the fluorescence 12 at the spots, 10, 10 ... in the plural through-holes 3 of the substrate 2.

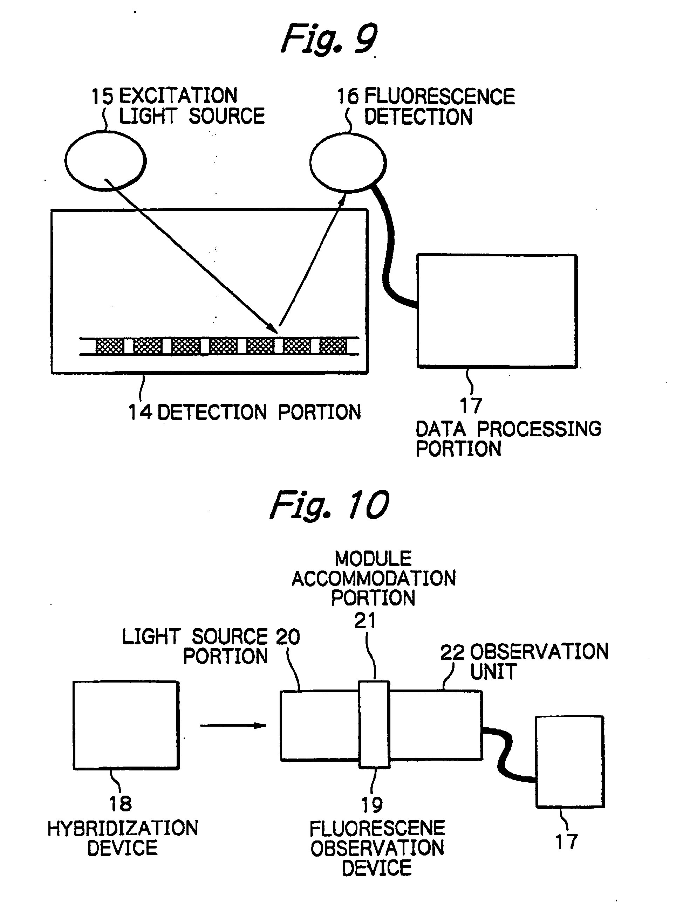

FIG. 9 is a conceptual view of a fluorescence detection system using the reaction probe chip of the present invention. In a detection portion 14, ultraviolet radiation from an excitation light source 15 is directed at the reaction probe chip, fluorescence emitted is subjected to fluorescence detection 16, and data on the detected fluorescence is transferred to a data processing portion 17 for processing of the data.

FIG. 10 expresses the conceptual view of FIG. 9 schematically as devices. This drawing shows a detection system in which hybridized spots obtained in a hybridization device 18 are observed for fluorescence by a fluorescence observation device 19 composed of a light source portion 20, a module accommodation portion 21, and an observation unit 22, and data obtained are processed by the data processing portion 17.

Next, the materials for and dimensions of the substrate, carrier, etc. described in the drawings are explained.

The reaction probe chip of the present invention can be prepared by a process comprising the steps of:
providing a substrate;
opening a regular array of discrete through-holes in the substrate that connect at least two of its surfaces;
fixing a carrier having a reactive surface, such as a powder of porous glass, a porous membrane or non-woven fabric, to at least one of the through-holes, which carrier bears on its surface a reacting substance such as DNA, RNA or PNA, antigen, antibody or epitope thereof, enzyme, protein or a functional fragment thereof.

Alternatively, the reactive substance may be carried on the surface of the carrier after fixing the latter to at least one of the through-holes.

The substrate may be a material unchanged and stable to the detection system, and needs to have surface characteristics suitable for fixing of the carrier. The preferred substrate is a glass substrate such as quartz glass or boro-silicate glass, or an inorganic substrate such as a silicon wafer. However, an organic substrate, such as a polyester film or a polyethylene film, can be used, if a method for bonding it to the carrier can be worked out. Suitable surface treatment can be applied to the surface of the substrate in order to adjust, for example, compatibility with a carrier binder.

In terms of its shape, the substrate is particularly preferably a flat plate, such as a film or a sheet. If the substrate is in the plate form, the thickness or size of the substrate is not restricted. The thickness of the substrate is determined, as desired, in consideration of shape stability required of the substrate. The size of the substrate is determined, as desired, in consideration of, for example, the number of the through-holes provided in the surface of the substrate.

The dimension of the plural through-holes regularly arranged in the substrate is not restricted, but is preferably 0.5 to 2 mm, particularly preferably about 1 mm.

The carrier is a material for bearing a reactive substance (probe molecule), such as DNA, RNA or PNA, antigen, antibody or an epitope thereof, enzyme, protein or a functional site polypeptide chain thereof. Preferred examples of the carrier are porous materials, such as a powder of porous glass, a porous membrane, and a nonwoven fabric. The shapes of the pores may be any shapes, as long as the porous material has a porous structure. Suitable surface treatment is preferably applied to the surface of the carrier in order to adjust, for example, compatibility with the reactive substance. The carrier in the through-hole should have a structure in which when a liquid is flowed during carriage of the reactive substance or during reaction for detection, the liquid can be flowed through the through-hole from top to bottom.

That is, the porous material, as the site of reaction, may be a material which fixes or grows the reaction probe molecule. There is no restriction on the type of the material, but porous glass powder or a glass fiber filter paper (nonwoven fabric) is preferred.

The method of carrying the reactive substance (probe molecule) onto the carrier need not be specified. However, any means usable for fixing can be used, such as a method which comprises fixing an amino group to the surface of the carrier, and then fixing a polypeptide chain with the use of glutaraldehyde. The reactive substance of a particular size need not be fixed and carried, but it is permissible to synthesize the reactive substance on the carrier as in the synthesis of oligonucleotide or oligopeptide, and use it unchanged.

The size and shape of the carrier can be selected arbitrarily. In view of the fact that the carrier bearing many various reactive substances is fixed onto the substrate, however, the preferred carrier is a powder with 1 to 100 microns, particularly preferably 3 to 20 microns. This is because a larger particle size is preferred in terms of the work efficiency of the process for carrying the reactive substance, but a smaller particle size is preferred when fixing the carrier after carriage of the reactive substance. The fixation of the carrier to the substrate is performed by dispersing the carrier in a solvent, such as water, together with a cellulose-based adhesive, such as cellulose nitrate, and arranging or printing the dispersion onto the substrate by means of a dispenser, spotter or the like.

The pore size of the porous glass powder or membrane is preferably 0.1 to 0.5 µm, and the fine gaps of the nonwoven fabric or filter paper are preferably several micrometers or less. Too small a pore size makes it difficult to filter the fluorescence labeled sample, so that a pore size of 0.1 µm or more is necessary.

The "reactivity" in the "reactive probe" described in the present invention refers not only to the properties of changing a chemical structure or the like ascribed to ionic bond or covalent bond by a chemical reaction, but also to the properties of bringing about binding to other substance by other mode, such as van der Waals force, hydrogen bond, coordinate bond, chemical adsorption, or physical adsorption. Examples of such a reactive probe are, but of course not restricted to, enzymes, antigens, DNA fragments, antibodies, epitopes, and proteins.

If an oligonucleotide, a DNA fragment synthesized on the carrier, is used as the reactive probe, for example, its hybridization with a DNA sample to be detected can result in the detection of DNA having a particular sequence.

Preparation processes for the reaction probe chip of the present invention, and a detection system using it will be described.

The reaction probe chip of the present invention has a structure in which the substrate is perforated with the through-holes and the reaction probes are fixed there (FIGS. 1 and 2(a) to 2(d)). At the site of reaction, such as the porous medium or nonwoven fabric, the probe molecules are fixed as shown in FIGS. 4(h) and 4(i).

The shape of the reaction site in the through-hole may include some cases, as shown in FIGS. 2(a) to 2(d). In any of these cases, the through-hole should be enough large to allow passage of a gene or the like to be analyzed, and should be designed to hold a reactive probe molecule.
1) With the substrate of such a structure, oligo-DNA's may be synthesized in many layers in situ and simultaneously, and used as reaction probe molecules. In this case, as shown in FIG. 5, the reaction probe molecules are synthesized at the same location in each of the through-holes of the probe chips stacked, whereby many chips with the same characteristics can be prepared at the same time. When the reaction chips are stacked and a liquid 13 is flowed from top to bottom through one through-hole after another as shown in FIG. 5, care should be taken to keep the respective through-holes liquid-tight so that the liquid does not pass through the clearance between the substrates to enter the laterally adjacent through-hole.
2) Using the holes that are similarly superposed one on another, cDNA's or the like can be carried in many layers simultaneously on the carriers. Thus, cDNA-fixed chips can be easily prepared.

For detection using the thus prepared chip, a sample of fluorescence labeled cDNA or the like is fed onto the reaction chip 5 fixed in the reaction cell 6 (FIG. 6(j)). The sample is slowly sucked to the opposite side, whereby the sample can be passed near the probe molecules (FIG. 6(k)). Thus, hybridization can take place easily (FIG. 6(l)). According to this procedure, the reaction occurs promptly and uniformly, and the device for hybridization is simplified.

The chip that has finished the reaction is detected, unchanged, by a conventional fluorescence detector (FIGS. 7 and 9). According this system, therefore, hybridization, detection and analysis are handled integrally (FIG. 10).

The present invention will be described in further detail by reference to the following examples, which in no way limit the invention.

### Example 1:

A quartz glass substrate (length 70 mm, width 25 mm, thickness 0.5 mm) having 10 x 5 regularly arranged through-holes of 1 mm in diameter was prepared. A powder of porous glass with a pore size of 100 nm and a particle size of 10 microns was filled into the through-holes, and baked. The upper and lower surfaces of the substrate were smoothed by polishing. The substrate was chemically cleaned, and then surface aminated using a silane coupling agent. Ten of the substrates were superposed, one on another, and different types of cDNA's were fixed to the respective through-holes by the customary method.

The substrates were placed in a reaction cell of polypropylene, and fluorescence labeled cDNA to be detected was poured into the reaction cell, and reacted. After reaction and washing, the chips were withdrawn from the cell, and analyzed by a fluorescence detector.

### Example 2:

A polyethylene terephthalate substrate (length 50 mm, width 30 mm, thickness 0.3 mm) having an arrangement of through-holes of 2 mm in diameter was prepared. A glass fiber filter paper was sandwiched between the substrates, and the composite was heat sealed to prepare a reaction chip substrate. One-hundred of the substrates were superposed, one on another, and reagents were sequentially passed through the respective holes vertically communicating with each other to synthesize different oligonucleotides.

The substrates were placed in a reaction cell of polypropylene, and fluorescence labeled cDNA to be detected was poured into the reaction cell, and reacted. After reaction and washing, the chips were withdrawn from the cell, and analyzed by a fluorescence detector.

### Example 3:

A Pyrex glass substrate (length 80 mm, width 30 mm, thickness 0.5 mm) having an arrangement of through-holes of 0.5 mm in diameter was prepared. A porous membrane comprising regenerated cellulose was pasted to the upper surface of the substrate. Separately, a powder of porous glass with a pore size of 100 nm and a particle diameter of 5 microns was prepared, and various oligonucleotides were synthesized by the customary method. These materials were dipped into the respective holes, and a dilute solution of cellulose nitrate, a cellulose-based adhesive, was added dropwise to fix the porous glass powder.

The substrates were placed in a reaction cell of polypropylene, and fluorescence labeled CDNA to be detected was poured into the reaction cell, and reacted. After reaction and washing, the chip was withdrawn from the cell, and analyzed by a fluorescence detector.

In all of the Examples, the hybridized spots emitted a strong fluorescence upon exposure to ultraviolet radiation. The sequences of DNA fragments in the DNA sample were elucidated by the fluorescence detector.

## Claims

1. A reaction probe chip comprising:
a substrate having a plurality of discrete, regularly arranged through-holes; and
a porous membrane or a non-woven fabric as a carrier being pasted to the substrate so as to close the through-holes, said carrier having probe molecules fixed thereto such that the probe molecules are different according to the through-holes, wherein the probe molecules are selected from the group consisting of DNAs, RNAs, PNAs and fragments thereof; oligonucleotides having arbitrary base sequences; antigens; epitopes of an antibody; antibodies; and enzymes, proteins and functional fragments of the enzymes or proteins.

2. The reaction probe chip of claim 1, wherein
the carrier having the probe molecules fixed thereto is a powder of porous glass, and
the powder of porous glass is entangled with or bound to the porous membrane or a non-woven fabric pasted to the substrate so as to close the through-holes.

3. The reaction probe chip of claim 1 or 2, wherein the substrate is a film or a sheet.

4. The reaction probe chip of claim 1 or 2, wherein the substrate is an organic substrate or an inorganic substrate.

5. The reaction probe chip of claim 2, wherein the powder of porous glass has particle size of from 1 to 100 micrometers.

6. A fluorescence detection system, comprising
- a reaction probe chip according to any of the preceding claims,
- an excitation light source (15), and
- a fluorescence detector (16).

## Patentansprüche

1. Ein Reaktionssondenchip, der Folgendes aufweist:
ein Substrat mit einer Vielzahl von diskreten, regelmäßig angeordneten hindurchgehenden Löchern; und
eine poröse Membran oder ein nicht gewebter Stoff als ein Träger, und
zwar aufgeklebt (pastenartig zum Anhaften gebracht) auf das Substrat um so die hindurchgehenden Löcher zu schließen, wobei der Träger Sonden- oder Probenmoleküle daran befestigt aufweist derart, dass die Sonden- oder Probenmoleküle unterschiedlich entsprechend den hindurchgehenden Löchern sind,
wobei die Proben- oder Sondenmoleküle aus der Gruppe ausgewählt sind, die Folgendes aufweist: DNAs, RNAs, PNAs und Fragmente davon; Oligonucleotide mit willkürlichen Basensequenzen; Antigene; Epitope eines Antikörpers; Antikörper und Enzyme, Proteine und funktionelle Fragmente der Enzyme oder Proteine.

2. Das Reaktionssondenchip nach Anspruch 1, wobei der Träger an dem die Sondenmoleküle befestigt sind, ein Pulver aus porösem Glas ist, und wobei das Pulver aus porösem Glas verwickelt oder verbunden ist mit der porösen Membran, oder einem nicht gewebten Stoff, aufgeklebt auf das Substrat um so die hindurchgehenden Löcher zu schließen.

3. Das Reaktionssondenchip nach Anspruch 1 oder 2, wobei das Substrat ein Film bzw. eine Schicht oder ein Flächenelement ist.

4. Das Reaktionssondenchip nach Anspruch 1 oder 2, wobei das Substrat ein organisches Substrat oder ein anorganisches Substrat ist.

5. Das Reaktionssondenchip nach Anspruch 2, wobei das Pulver aus porösem Glas eine Teilchengröße von 1 bis 100 Mikrometer besitzt.

6. Ein Fluoreszenzdetektionssystem, welches Folgendes aufweist:
- ein Reaktionssondenchip gemäß einem der vorhergehenden Ansprüche,
- eine Anregungslichtquelle (15), und
- einen Fluoreszenzdetektor (16).

## Revendications

1. Sonde-puce réactive comprenant :
un substrat comprenant une pluralité de trous traversants discrets, agencés de manière régulière ; et
une membrane poreuse ou une étoffe non tissée en tant que support qui est encollée sur le substrat de façon à fermer les trous traversants, des molécules sondes étant fixées audit support de telle sorte que les molécules sondes sont différentes selon les trous traversants, où les molécules sondes sont choisies dans le groupe constitué d'ADN, d'ARN, d'ANP et de fragments de ceux-ci ; d'oligonucléotides ayant des séquences de base arbitraires ; d'antigènes ; d'épitopes d'un anticorps ; d'anticorps ; et d'enzymes, de protéines et de fragments fonctionnels des enzymes ou des protéines.

2. Sonde-puce réactive selon la revendication 1, dans laquelle
le support auquel sont fixées les molécules de sonde est une poudre de verre poreux, et
la poudre de verre poreux est enchevêtrée avec ou liée à la membrane poreuse, ou à une étoffe non tissée encollée sur le substrat de façon à fermer les trous traversants.

3. Sonde-puce réactive selon la revendication 1 ou 2, dans laquelle le substrat est un film ou une feuille.

4. Sonde-puce réactive selon la revendication 1 ou 2, dans laquelle le substrat est un substrat organique ou un substrat inorganique.

5. Sonde-puce réactive selon la revendication 2, dans laquelle la poudre de verre poreux a une taille de particule de 1 à 100 micromètres.

6. Système de détection de fluorescence, comprenant
- une sonde-puce réactive selon l'une quelconque des revendications précédentes,
- une source de lumière d'activation (15), et
- un détecteur de fluorescence (16).
